# EUROPEAN PATENT APPLICATION

(11) **EP 2 550 925 A1**
(43) Date of publication of application: **30.01.2013**
(21) Application number: 11382259.7
(22) Date of filing: 28.07.2011
(51) Int. Cl.: A61B 18/20

(54) **Multipurpose portable laser depilationdevice**

(71) Applicant: Gomez de Diego, Eduardo Antonio, 28231 Las Rozas (Madrid) (ES)
(72) Inventor: Gomez de Diego, Eduardo Antonio, 28231 Las Rozas (Madrid) (ES)
(74) Representative: Temino Ceniceros, Ignacio

(57) **Abstract**

Portable laser hair removal of multi purpose use comprising a housing (2) with a head (11) and transparent dome (3) with bottom hole (4) having a laser illumination system with two diodes (5, 6), different in wavelength, power, opening, pulse period and average power pulse size in red and infrared, respectively.

## Description

The present invention is directed to an apparatus designed for laser hair removal, with domestic character, portable and easy to use for the user. The device is configured as an instrument of small size, low economic cost due to the use of standardized components and their structural simplicity. The device also has the particularity of having one, two or even three types of different laser light illumination incorporated simultaneously or independently, so that one may be more suitable for light skins and light hair, another more suitable for olive skins and dark hair and other for black skins and dark hair, being selected by the user depending on the skin and hair typologies, thus having a multipurpose nature between different users and types of skin and hair. The field of application of the present invention is the industry dedicated to the manufacture of laser hair removal apparatus.

### State of the prior art

The scientific basis of laser hair removal is the principle of selective photothermolysis, which predicts that a selective thermal injury of a pigmented structure will be produced when a sufficient luminous flux at a given wavelength (absorbed by the target) for a less or equal time that the thermal relaxation time of the target is deposited.

Laser hair removal was first described in 1996. Complications are rare if the recommended treatments are followed, the only more common side effect being the temporary change in pigmentation.

The basic principles of laser hair removal consist of that the light incident on a tissue can be transmitted, reflected, absorbed or scattered, depending on the tissue composition and the wavelength (color) of light.

Each type of tissue in the human body has an absorption coefficient that depends on its specific absorbent components, also called chromophores.

The main chromophores of the human body are four: hemoglobin (blood), melanin (skin, hair, moles), water (in all biological tissues) and proteins (scattering center: covalent bonds present in the tissues).

When light is absorbed by a chromophore, their reaction (destruction, vaporization, reduction or deactivation) depends on light intensity and exposure time. In the case of photo hair removal, the melanin containing the hair follicle is used as a luminous energy-absorbing chromophore. The hair follicle is part of the skin that gives growth to the hair by concentrating the stem cells.

The light applied can be of two types:
- Laser: coherent, monochromatic, collimated (ruby, alexandrite, diode, Nd: YAG)
- IPL: non-coherent, polychromatic not collimated (Intense Pulsed Light, Xenon lamps with long-pass filters of different cutoff wavelengths between 590 and 1200 nm).

The fact that the laser light is monochromatic (only emits in a wavelength) makes has a selective effect in its application on a tissue and, according to different studies, makes it ideal for hair removal treatments on different skin types. The IPL systems emit at a wider band of wavelengths, making the treatment less effective and more risky for people with dark skin.

On the other hand, the application of light on the skin may destroy hair follicles in three different ways: thermal (by local heating), mechanical (by shock waves or violent cavitation) and chemical (by generation of toxic compounds), all of these can be found in the literature and have been studied for hair removal.

According to the studies reviewed the most effective and less risks is the photothermal effect, also called selective photothermolysis, process by which the transfer of laser energy is restricted to a particular area due to the selective absorption of a chromophore in this specific area. This means that by choosing the wavelength and exposure time only the chosen target is damaged.

Likewise, it must bear in mind that the effectiveness of laser hair removal depends largely on individual skin phenotype, specific hair color, hair type or texture, density, hormonal factors and anatomical localization, although it seems demonstrated that all laser systems temporarily reduce hair growth in all types of color (except gray hair), and existing, however, a close relationship between hair color and the luminous flux used in the permanence of hair removal.

Currently, the most frequently used lasers for portable laser hair removal portable equipments are semiconductor lasers (laser diodes) of 500 mW at 808 nm with focusing optics, since its wavelength allows good depth of penetration and absorption of melanin in the follicle, in addition, the current technology makes available high-power diodes, which allows a large area of the beam exit to accelerate the treatment, allows to have laser diodes at different wavelengths to cover a wider range of types of hair and skins, being a very compact system that has very low energy.

Logically, then, in this field are known many types of laser hair removal apparatus and devices, among which it is noteworthy as the most relevant, the following patents:
- the document US 5,836,938 describes a system designed with an invasive needle that is inserted into the hair root and there emits radially luminous energy so as to vaporize the hair. It proposes the use of topical solutions enhancing the damage. The system is complex and annoying to use for the user as well as impractical, painful and slow.
- The document WO 99/04711 describes a pulsed CO₂ laser (10600 nm) with spot size of 1 cm diameter with a topical solution of charcoal suspension in oil peach, what is outside the desired application for the apparatus described herein. This document also proposes the use of lasers emitting near-infrared (1060 nm) with a spot diameter of 4 to 6 mm and very low doses (3-6 mJ/cm²), so low that it is doubtful that it can function as a depilatory.
- The document WO 99/60937 describes a pulsed laser of 25 mW and 0.1 sec in near-infrared (900-1300 nm) with penetration of 1 cm and transported through an optical fiber. According to this publication, the lipid components of sebum (contained in the hair follicle and hair cover) absorb very well at these wavelengths, producing heat and damage due to the heat conduction to the papilla, where blood vessels and tissue around the papilla are coagulated, thus preventing the hair from growing back. The suggested dose in this patent is small enough to produce lasting effects in addition to the hair is very thermally conductive, so that radiation losses before the light reaches the papilla are large by reducing the effectiveness of the device.
- The document US 6,168,589 claims the use of any coherent light source (laser) between 694 and 900 nm for hair removal, with a treatment system of two or more high power (10-40 j/_{CM}²) long pulses (2-30 msec) separated by a period of 10-30 msec and a spot size greater than or equal to 10 mm. The light generated by the laser is transported to the surface of the skin via optical fiber, thus changing the irradiance profile of the treated area. This patent proposes an apparatus that it must be manipulated by an operator without any type of contact sensor and with different operation options that should be adjustable depending on skin/hair of the patient, making it difficult for their understanding and extrapolation for a use at home.
- The document WO 01/45795 uses a minimum of 5 laser sources focused on a same point, which is conducted through a diffuser cylinder to the surface of the skin. The patent claims that this method is not harmful in any way for the surface of the skin, but what it does not show is that it can hardly exploit the qualities of penetration and concentration of laser light energy by passing it through a diffuser and breaking its spatial coherence. This process also involves the use of more powerful lasers, resulting in higher cost of final product.
- The document WO02/096311 describes a protection system for the application of laser light for hair removal, based on an extension-replicating mechanical system by which it is difficult for the application head is launched if not intentionally. It also includes a proposal of pre-programmed sequence for laser hair removal treatment, very broad and ambiguous to comprise all the possible lasers, pulse sizes and times. The security system design is complex and costly when implemented in series production, and does not cover portable, but only to the application area which includes a diffuser tube which reduces the final power of the laser, i.e., is not the laser itself, as is desirable.
- The document US 6,632,218 describes a system containing an alexandrite laser with an optical fiber carrying the laser light generated to the surface of the skin with the opening of the optical fiber. It utilizes pulses greater than 1 msec for treatment, fluencies of 10-50 J/cm², as well as a topical solution to mark the spots on the skin to be treated and to prevent harmful reflections in it. The proposed system is quite bulky, little portable and definitely has to be handled by a professional. It has no security control system and uses an alexandrite laser that one of the less efficient system for this application.
- The document US 2004/015156 describes a device with laser illumination which includes a colorimeter to determine the color of the skin and choose from different doses to optimize the performance. Exposure of experimental measurements carried out with 62 users in which is established a correlation between skin type according to Fitzpatrick scale and dose of laser. In this document, generally very technical, it is claimed the method of application more than a specific product.
- The document US 2005/0256514 proposes the use of at least 5 packages (0-5 to 10 msec of duration) of pulses (on the order of nanoseconds in size) in the administration of the dose (2 to 60 J/cm²), rather than the total dose deposited in a single pulse to avoid skin damage.
- The document W02007/034161 is directed to the domestic sector, and has a safety sensor that classifies it as cone Class I, however, it is not portable because contains a control table and a head (phone type) carrying the applicator.
- US Patent 7,250,045 where is proposed a lightweight (<1 kg) apparatus without cable and the use of a diffuser to the beam output, to avoid the possibility that the laser causes eye damage, as well as the inclusion of a contact sensor. Due to the variation of the power of a laser diode with the use (battery wear), the power decreases very fast and therefore the portability of equipments based on battery power is not realistic. On the other hand, as seen above, placing an optical diffuser at the output of a laser beam reduces its power, its focalization and destroys its spatial coherence, which reduces the penetration distance into the tissue.
- US 7 329 252 proposes a semiconductor laser emitting at a wavelength between 600 and 1600 nm with powers between 5 and 1500 mW, which includes a system that can expel air (cooling) or absorb it (moving the area to be treated closer to the point of laser emission).

It is proven, therefore, that coexist in the market many types of apparatus and devices for laser hair removal, which, however, in most cases consist of complex and expensive equipments designed for professional use and should be handled by staff experienced or, in the case of any of them has portable character, having a very limited range of application that greatly restricts the target customer to which can be directed, which, further, not have an optimal effectiveness in the obtained results.

On the other hand, the device US 2005/154382 describes a dermatological treatment device of illumination and collection of information from the patient's skin, which may also comprise at least one laser element in undertaking various dermatological treatments. Indeed, this document describes in general the use of lasers, but for a certain type of treatment in general and without specifying the function of said lasers, if they are equal to each other or different, as well as its primary task. The laser used in this document is Nd: YAG laser, which has serious drawbacks, such as the low melanin absorption, its lack of effectiveness for use in fine and light hair, and is also an expensive and large. Finally, this type of lasers requires a large electrical power for its operation, making unviable frankly its use in a portable apparatus, in addition to require a large treatment area to function properly, which also redounds to their inability to use in a portable device. It is logical in this document using this type of laser, because it is used primary to illuminate areas of operation by a dermatologist and optionally incorporate means for treatment, including a hypothetical hair removal.

In this document also is described the use of laser diodes, but only for the illumination of the treatment areas of the skin that are displayed, as this constitutes the fundamental objective of the patent. The presence of these lighting elements, complementary to the discussed laser treatment, allows increasing the contrast in the visualization of the target area. This use can also allow that the visualization systems are used as tomographic devices or high resolution microscopy systems. In any case, the skin treatment is always carried out using an Nd: YAG type laser.

Finally, WO 2004/075681 describes a hair remover device comprising a contact sensor that serves to prevent the turning on of the laser diodes in positions other than those of treatment. This document can use up to two arrays of identical lasers, without delimited functions. These laser sources are focused on a same point, which is conducted through a diffuser to the surface of the skin, therefore, can hardly exploit the qualities of penetration and concentration of laser light energy by passing it through a diffuser and breaking its spatial coherence. This process also involves the use of more powerful lasers, resulting in higher cost of final product. Specifically, in this document is indicated that the use of laser diodes is rejected due to have low power and specifically prefer the use of laser diode arrays, i.e. a plurality of laser diodes concentrated in a single package and with a single diffuser, which necessarily have a higher power consumption, greatly reducing the autonomy of the portable device. Finally, this document rejects the use of light sources (laser diode arrays) with a spot less than 5 mm, resulting also in greater electrical power to be employed and, therefore, less autonomy.

Taking the documents US2005/154382 and W02004/075681 as background closer to the invention described herein, one skilled in the art would have to solve various technical problems that make this combination is not obvious.

It is arguable, therefore, that the applicant has not found any other portable laser hair removal apparatus of multi purpose use or has technical, structural and constitutive characteristics similar to that described in the first claim of this specification.

### Explanation of the invention

The portable laser hair removal of multi purpose use, object of the present invention meets the objectives indicated, such as have an optimized portable hair removal apparatus, user-friendly, so it can be used by a normal user and exclusively by a professional, low operating and acquisition costs, so that is accessible to all customers, regardless of its hair color, classified as Class I of laser safety according to the standard of quality UNE-EN 60825-1 and high level of effectiveness in hair removal.

Therefore, and concretely, the apparatus of the invention is of the type that is made from an ergonomic housing and has an illumination system of coherent laser light, suitable to be absorbed by melanin with deep penetration in tissues of the user in order to achieve the hair fall, and comprising a housing, front part of which extends into a head covered by a transparent dome provided with a hole at the bottom, and inside of which have laser illumination means, constituted by, at least, one laser diode of average power in the red and near infrared, activated by actuation means, which in turn comprise a plurality of touch buttons with a plurality of pulse positions, at least one by a laser diode comprising the apparatus and a releasing position, said positions being materialized in corresponding touch buttons; and characterized in that, comprises, at least, a contact resistive sensor calibrated for the detection of human skin, so that when it detects that the apparatus is placed on the skin or scalp, enables the shot of the laser diode previously selected by pressing a shot launcher touch button; and where the diode incorporates an optical collimator lens, at least one by said laser diode, made of acrylic material and anti-reflective coating that focuses the laser into the hole at the bottom end of head.

An important advantage of the invention is that the proposed apparatus does not comprise protecting windows beyond the lens that is placed next to the laser, a transparent dome to position the apparatus and with the appropriate length to facilitate the application of the laser on the hair to be removed.

The fact of using optics with anti-reflective coating minimizes reflection, absorption and scattering losses on surfaces and allows the use of laser diodes farther from the scalp, making use of this circumstance to adjust the output size of the beam at a lower power, resulting in lower power consumption and greater autonomy of the battery that powers the apparatus.

Since the use of monochromatic laser light in the range of 650 to 900 nm is the most effective permanent hair removal mode known, the advocated apparatus contemplates the use of laser diode (LD) that can be available in the market in different output wavelengths and are easily controlled by simple electronics to emit the desired pulse in each case. Thus, hair removal equipment can be manufactured with different output wavelengths by simply choosing another laser diode with different wavelength but the same electronic activation circuit.

Do not confuse a laser diode with an array of laser diodes (referred to as laser diode bars in the state of the art). The arrays typically consist of between 20 to 50 laser diodes, which are consuming more energy, and more power to penetrate the skin, but for that reason, are not valid in portable applications. However, the diodes chosen have a good quality/price ratio, although emit with a large beam divergence (which is not true, of course, with arrays of laser diodes). This last feature makes the output of each laser beam is elliptical, so it includes the use of lenses to concentrate the beam in an area much more convenient.

Specifically, the diodes used, depending on whether is a particular embodiment of the apparatus for one, two or three LEDs are as follows:
- For olive skin and dark hair, power of 500 mW and wavelength at 808 nm, with collimator lens with focal of 18 mm in length and effective diameter of 4.37 mm, such as AlGaAs by *Roithner Lasertechnik.*
- For light skin and light hair, power of 50 mw and wavelength at 685 nm, with collimator lens with focal of 18 mm in length and effective diameter of 4.37 mm, such as InGaAIP by *Roithner Lasertechnik.*
- For black skin and dark hair, with power of 350 mw and wavelength at 1064 nm, with collimator lens with focal of 18 mm in length and effective diameter of 4.37 mm, such as AlGaAs by *Roithner Lasertechnik.*

Logically, since there are included one, two or three diode lasers at three different wavelengths for three types of different skin/hair, with different powers and openings, each must be pressed with a different period (time between pulses) and different pulse size.

For example, in the case of equipment with two outputs, the two lasers are located at a distance of 36.76 mm from the opening (elliptical with major and minor axes of 4 mm and 3 mm, respectively) and each laser is separated from the vertical 9° (18° between the two lasers). At this distance, and given the elliptical laser output, the collimator lens cited above should be used for laser with focal of 18 mm and effective diameter of 4.37 mm has been noted before.

The hair removal apparatus has been designed so that there is a strong absorption by melanin and deep penetration into the tissues, by which is configured as a system capable of producing injuries in the retina. Due to this risk of eye injury, it is provided for the use of a contact sensor that prevents the turning on of the laser diodes in positions other than treatment, thus making the instrument becomes classified as Class I according to the Standard of Quality UNE-EN 60825-1, that is, safe in all reasonably foreseeable conditions, allowing to consider the instrument as commonly used in home.

This system does not alter the specifications of the laser illumination (spatial and temporal coherence, opening, power), but only when it is applied, unlike the alternative used in other apparatus known in the market, which consists of placement of a diffuser in front of the output laser beam which makes breaking the spatial coherence of the laser and its danger is eliminated.

The modification of the spatial coherence of laser beam, has the disadvantage of reducing the laser penetration into the tissues, being thus necessary to increase the base power of the laser (increasing system cost and consumption of the device, reducing its autonomy), modifying the opening of the laser beam making larger in diameter, so reducing the effective dose to the tissues, being necessary to use a more powerful laser. Also changes the output power of the beam as being a refractive element, the light has to pass through the diffuser, which always absorbs and reflects part of incident light.

Following the invention, it is noteworthy that the device has an actuation system based on touch buttons or "touch control" (TC), instead of mechanical or membrane (more conventional) buttons, which, as known, is based on the physical phenomenon of load variation of a capacitor due to the electric charge of the human body.

It should be noted that the structural configuration of the apparatus influences the way of working of CT as shown in the sensitivity with which the device will work. The electrode is electronic element of the system more dependent on instrument design.

Thus, the size of the TC electrode should have an average ratio defined by its smallest dimension from 4 to 1 regarding to the distance of the same to the contact point of the finger with the surface of the apparatus, being able to take many shapes (circular plane, rectangular plane, spring-like...).

Regarding the operation of the instrument, it must say that is very simple, containing as many pushing positions as diodes are in the apparatus (a push-button for each diode laser used), although the contact sensor acts as a third (automatic laser) button when it detects that the instrument is positioned on the skin/scalp.

Depending on the number of lasers that the equipment includes according to the model, there may be various operation options such as, for example, the following:
- Embodiment with a laser: turning on of the laser, turning off of the laser.
- Practical embodiment with two lasers: in all possible combinations that permit those of the type (a) 685 nm, (b) 808 nm:
   o Turning on of the laser 685 nm and no operation of the laser 808 nm.
   o Turning on of the laser 808 nm and no operation of the laser 685 nm
- Practical embodiment with three lasers:
   o Turning on of the laser 685 nm and no operation of the laser 808 nm or 1064 nm.
   o Turning on of the laser 808 nm and no operation of the laser 685 nm or 1064 nm.
   o Turning on of the laser 1064 nm and no operation of the laser 808 nm or 685 nm.

With the apparatus described the main objective is achieved that is a hair removal apparatus with the greater effectiveness possible by the use of at least one laser diode, with a maximum safety for use by non-specialists, with the inclusion of contact sensor, multi purpose, by the use of different lasers at once, and portable, by the fact of using a electronics and a associated structure which allows to minimize power consumption without affecting the normal operation.

Throughout the description and claims the word "comprise" and its variants are not intended to exclude other technical features, additives, components, or steps. For those skilled in the art, other objects, advantages and features of the invention will become apparent in part of the description and part of the practice of the invention. The following examples and drawings are provided by way of illustration and are not intended to be limiting of the present invention. Furthermore, the present invention covers all the possible combinations of particular and preferred embodiments listed here.

### Brief description of the drawings

To complement the description that is being made and in order to help a better understand the characteristics of the invention, this specification is accompanied by, as an integral part thereof, a set of planes, in which with illustrative and not limitative character, the following has been represented:
Figure 1 shows a top view of the multi-purpose hair removal apparatus object of the invention, wherein Fig. 1a shows an embodiment with two diodes, while Fig. 1b shows an embodiment with a single laser diode.
Figure 2 shows a side view of the multi-purpose hair removal apparatus object of the invention, wherein Fig. 2a shows an embodiment with two diodes, while Fig. 2b shows an embodiment with a single laser diode.
Figure 3 shows an elevation view of the multi-purpose hair removal apparatus object of the invention, wherein Fig. 3a shows an embodiment with two diodes, while Fig. 3b shows an embodiment with a single laser diode.
Figure 4 shows a sectional view, according to a cross section of the apparatus part corresponding to the model with two laser diodes (Fig. 4a) or a laser diode (Fig. 4b), according to the invention, in which the diodes that incorporates are housed, appreciating in it the optical arrangement thereof.

### Detailed description of the embodiments

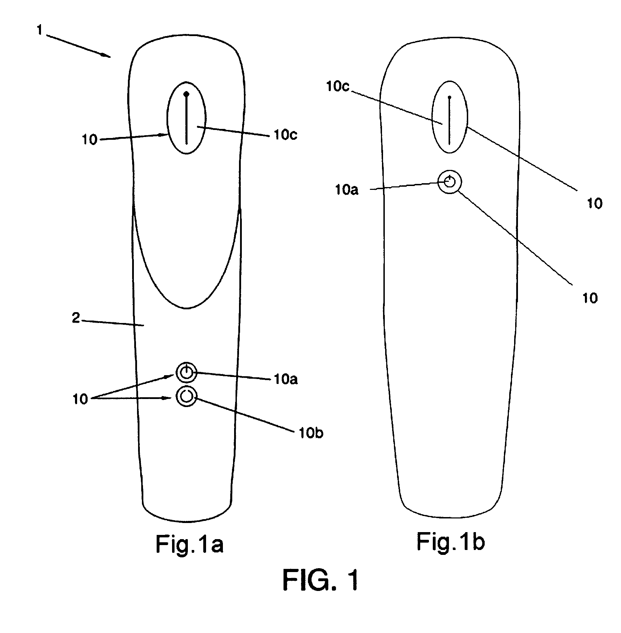

As shown in the accompanying figures, the exemplary apparatus (1) corresponding to the embodiment with two lasers (Fig. 1) in question is made from an ergonomically designed housing (2), approximately long-shape, front part of which extends into a head (11) covered by a transparent dome (3) provided with a hole (4) at the bottom, and inside of which have laser illumination means constituted by, at least, one laser diode (5, 6) of different wavelengths, power and opening, each pressed with different period (time between pulses) and different pulse size, which are optimized one for dark hair (5) and other for light hair (6) with average power in red and infrared, respectively, through an optical lens (7) made of acrylic material and anti-reflective coating that focuses the laser into the hole (4) at the bottom end of head (11).

In this particular example, the aforementioned diodes are preferably:
- For dark hair, diode with a power of 500 mW and wavelength at 808 nm, with collimator lens with focal of 18 mm and effective diameter of 4.37 mm.
- For light hair, diode with a power of 50 mw and wavelength at 685 nm, with collimator lens with focal of 18 mm and effective diameter of 4.37 mm.

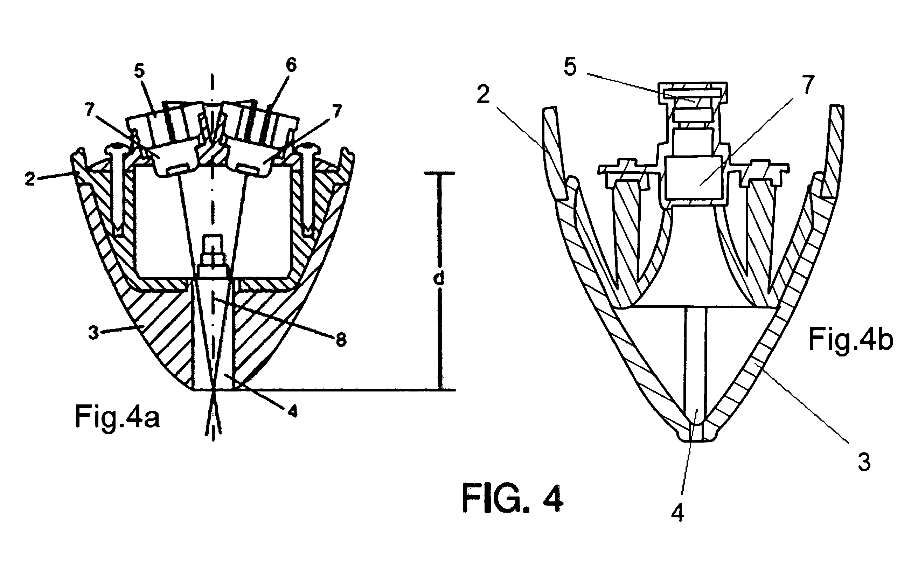

Furthermore, as shown in Figure 4, the two lasers, i.e., the diode array (5, 6) and corresponding lenses (7), are located at a distance (d) of 36.76 mm from the opening or hole (4) and each laser is separated from the vertical axis (8) 9°, having a distance between both of 18º. At this distance, and given the elliptical laser output, it should be used a collimator lens (7) for laser with focal of 18 mm and effective diameter of 4.37 mm previously noted.

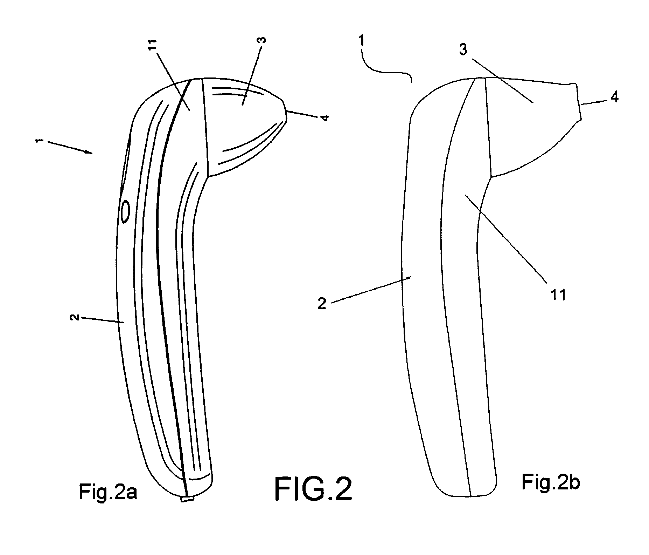

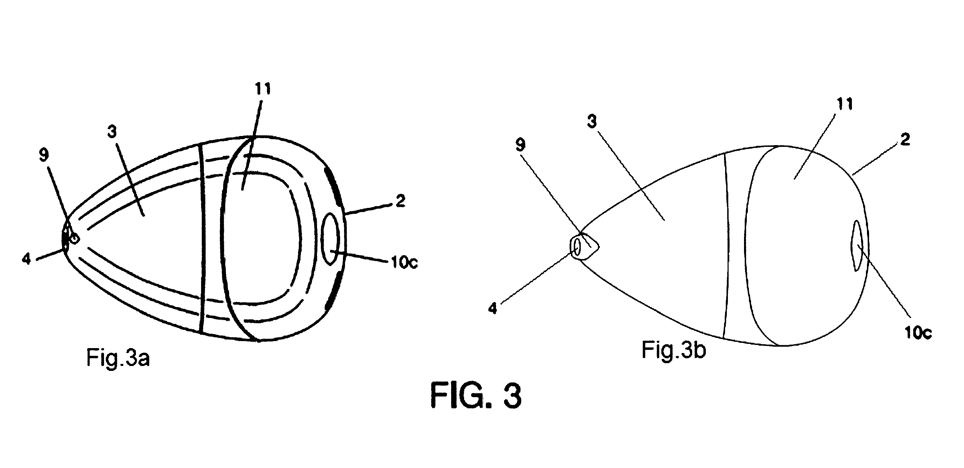

In the embodiment with a laser diode (Fig. 1b, Fig. 2b, Fig. 3b, Fig. 4b) is made from an ergonomically designed housing (2), approximately long-shape, front part of which extends into a head (11) covered by a transparent dome (3) provided with a hole (4) at the bottom, and inside of which have laser illumination system constituted by a single laser diode (5), of different wavelengths, power and opening, each pressed with different period (time between pulses) and different pulse size, which are optimized one for dark hair and light hair, depending on what it is desirable from factory, since unlike the embodiment with two diodes, in this case it will be implemented one of the diodes mentioned above through an optical lens (7) made of acrylic material and anti-reflective coating that focuses the laser into the hole (4) at the bottom end of head (11).

The apparatus, in order to prevent a possible eye injury, includes a contact resistive sensor (9) that prevents the turning on of the lasers, both in the embodiment with one or two diodes, in positions other than the treatment, for which is calibrated to detect the skin, being able to differentiate it from other materials, being preferably a sensor specifically designed to be as small and economical as possible.

Finally, the apparatus has an actuation system based on touch buttons or "touch control" (TC), and comprising a electrode, a detection chip, a interpretation logic, resistors, capacitors and wiring, with the electrode of a smallest dimension of 4 to 1 with respect to the distance of the same to the touch button (10) on the surface of the device, button that can take many shapes (circular plane, rectangular plane, spring-like...).

As noted in paragraphs above, the operation of this example of apparatus corresponding to the model with two lasers (Fig. 1a) is very simple comprising two pushing positions, with corresponding touch buttons, one for dark hair diode (10a) and other for the one of light hair (10b) and a shot launcher (10c), operating the contact sensor (9) as a third automatic button of laser when it detects that the instrument is positioned on skin/scalp.

Thus, pressing the dark hair touch button (10a), the dark hair diode (5) at 808 nm is connected but not turned on. However, placing the head (11) on the treatment area, the contact sensor (9) detects the skin/scalp and the laser is prepared to emit, which will only begin the emission when the touch button of the shot launcher (10c) is pressed.

Logically, in the embodiment with a single laser diode (Fig. 1b), the operation procedure or apparatus operation will be as described, activating the laser, by placing the head until the contact sensor (9) detects the skin and emitting only when the shot launcher (10c) is actuated.

Pressing the light hair touch button (10b), the light hair diode (6) at 685 nm is connected but not turned on, while placing the head (11) on the area to be treated, the contact sensor (9) detects the skin/scalp and the laser is prepared to emit. Pressing the touch button of the shot launcher (10c) the selected laser starts to emit.

Similarly, logically, in the embodiment with three diodes, a third touch button will be incorporated, which will act as those described.

Thus, the lasers are operated only when the apparatus is turned on, the contact sensor detects that the instrument is positioned on skin or scalp and the launcher is pressed.

The electronics of the apparatus is optimized for use in a portable apparatus and comprises at least a microcontroller that manages both the battery charge and the operation of lasers, the contact sensor (9) and the touch buttons (10, 10a, 10b, 10c) according to the operation described above. Similarly, activation of the lasers (5, 6) is performed not directly with the microcontroller, but through an activation circuit and current increase, which converts the signal from the microcontroller into an electrical signal (pulse) with a suitable current for the diodes (5, 6).

Additionally, it has provided a buzzer connected to the microcontroller, so that under certain conditions, the apparatus can warn the user not only visually, for example, with LEDs indicating the activity of the apparatus, but also acoustically, for example, when the battery is low. The power of the apparatus is via a standard rechargeable battery, having the appropriate circuitry for it.

## Claims

1. Portable laser hair removal of multi purpose use, of the type that is made from an ergonomic housing (2) and has an illumination system of coherent laser light, suitable to be absorbed by melanin with deep penetration in tissues of the user in order to achieve the hair fall, and comprising a housing (2), front part of which extends into a head (11) covered by a transparent dome (3) provided with a hole (4) at the bottom, and inside of which have laser illumination means constituted by, at least, one laser diode (5, 6) of average power in the red and near infrared, activated by actuation means, which in turn comprise a plurality of touch buttons (10, 10a, 10b, 10c) with a plurality of pulse positions (10a, 10b), at least one by a laser diode (5, 6) comprising the apparatus and a releasing position (10c), said positions being materialized in corresponding touch buttons; and **characterized in that**, comprises, at least, a contact resistive sensor (9) calibrated for the detection of human skin, so that when it detects that the apparatus is placed on the skin or scalp, enables the shot of the laser diode (5, 6) previously selected by pressing a shot launcher touch button (10c); and where the diode (5, 6) incorporates an optical collimator lens (7), at least one by said laser diode (5, 6), made of acrylic material and anti-reflective coating that focuses the laser into the hole (4) at the bottom end of head (11).

2. Apparatus according to the claim 1 wherein the laser diode (5, 6) is at least a laser diode (5, 6) selected from: (a) a first diode optimized for dark hair (5), with power of 500 mW and wavelength at 808 nm; (b) a second diode for light skin and light hair (6), with power of 50 mw and wavelength at 685 nm, and (c) a third diode for black skin and dark hair, with power of 350 mw and wavelength at 1064 nm.

3. Apparatus according to the claim 1 and 2 wherein the laser diode (5, 6) and its corresponding collimator lens (7) is located at a distance (d) of 36.76 mm of the opening or hole (4) and each laser is separated from the vertical axis (8) 9°, with a distance between the lasers (5, 6) of 18º.

4. Apparatus according to the claims 1 to 3 wherein the collimator lens (7) for laser incorporating the diode (5, 6) has a focal of 18 mm and effective diameter of 4.37 mm.

5. Apparatus according to the preceding claims, wherein comprises at least one touch button selected from: one for the dark hair diode (10a) and other for the one of light hair (10b).

## Amended claims

### Amended claims in accordance with Rule 137(2) EPC.

**1.** Portable laser hair removal of multi purpose use, of the type that is made from an ergonomic housing (2) and has an illumination system of coherent laser light, suitable to be absorbed by melanin with deep penetration in tissues of the user in order to achieve the hair fall, and comprising a housing (2), front part of which extends into a head (11) covered by a transparent dome (3) provided with a hole (4) at the bottom, and inside of which have laser illumination means constituted by, at least one laser diode (5, 6) and up to three laser diodes, of average power in the red and near infrared, activated by actuation means, which in turn comprise a plurality of touch buttons (10, 10a, 10b, 10c) with a plurality of pulse positions (10a, 10b), at least one by a laser diode (5, 6) comprising the apparatus and a releasing position (10c), said positions being materialized in corresponding touch buttons; and the portable laser hair removable comprises, at least, a contact resistive sensor (9) calibrated for the detection of human skin, so that when it detects that the apparatus is placed on the skin or scalp, enables the shot of the laser diode (5, 6) previously selected by pressing a shot launcher touch button (10c); and the portable laser hair removable is **characterized in that** the diode (5, 6) incorporates an optical collimator lens (7), at least one by said laser diode (5, 6), made of acrylic material and anti-reflective coating, that focuses the laser into the hole (4) at the bottom end of head (11).

**2.** Apparatus according to the claim 1 wherein the laser diode (5, 6) is at least a laser diode (5, 6) selected from: (a) a first diode optimized for dark hair (5), with power of 500 mW and wavelength at 808 nm; (b) a second diode for light skin and light hair (6), with power of 50 mw and wavelength at 685 nm, and (c) a third diode for black skin and dark hair, with power of 350 mw and wavelength at 1064 nm,

**3.** Apparatus according to the claim 1 and 2 wherein the laser diode (5, 6) and its corresponding collimator lens (7) is located at a distance (d) of 36.76 mm of the opening or hole (4) and each laser is separated from the vertical axis (8) 9º, with a distance between the lasers (5, 6) of 18º.

**4.** Apparatus according to the claims 1 to 3 wherein the collimator lens (7) for laser incorporating the diode (5, 6) has a focal of 18 mm and effective diameter of 4.37 mm.

**5.** Apparatus according to the preceding claims, wherein comprises at least one touch button selected from: one for the dark hair diode (10a) and other for the one of light hair (10b).
